# EUROPEAN PATENT SPECIFICATION

(11) **EP 0 285 585 B1**
(45) Date of publication and mention of the grant of the patent: **21.05.1997**
(21) Application number: 88850091.5
(22) Date of filing: 17.03.1988
(51) Int. Cl.: A61G 9/00, A61F 5/44

(54) **A device for collecting and temporarily storing urine**
Vorrichtung zum Sammeln und vorläufigen Aufbewahren von Urin
Dispostif pour collecter et garder temporairement de l'urine

(30) Priority: 30.03.1987 SE 8701299
(43) Date of publication of application: 05.10.1988
(73) Proprietor: Nilsson, Leif, S-260 40 Viken (SE)
(72) Inventor: Nilsson, Leif, S-260 40 Viken (SE)
(74) Representative: Andersson, Andy Rudy

(56) References cited:
- EP-A- 0 209 508
- WO-A-82/01648
- FR-A- 1 230 233
- FR-A- 2 291 428
- GB-A- 2 088 023
- US-A- 3 928 875

## Description

### Field of invention

The present invention relates in general to a disposable device for collecting and temporarily storing urine of an individual of either sex, comprising a tube having urine inlet and an opposite located urine outlet, and a urine collecting bag having an inlet end and an openable and closable outlet end and a non-return valve located between said urine tube and said collecting bag, and serving as a connector means between said urine tube and said collecting bag, said urine tube tapering towards said valve means, wherein a proximal end of valve means communicates with the urine tube, and a distal end communicates with the urine collecting bag, wherein the distal end of the valve body is embraced by the upper open end of the bag to establish communication between the valve means and said bag, wherein the valve means together with the urine collecting bag form a unit, wherein the outer surface of the distal end of the valve body lies sealingly against the inner wall of the urine tube in the region of its second opening.

### Background prior Art

Various devices for collecting urine discharging from a patient, e.g. a bed-ridden patient or persons who suffer from incontinence are known to the art. The most common of these devices comprise glass urine bottles or urine bottles that are made of a plastics material which is resistant to uric acid. These known devices, however, are expensive to purchase and must be sterilized each time after use. Patients who are required to lie in an incumbent position may find it difficult to manipulate devices of this kind. Furthermore, when the patient has finished using the bottles, the bottle must be removed from the bed and the contents of the bottles emptied by ward staff at the earliest opportunity. In the case of hospital patients who are obliged to lie on their stomachs, the matter of urinating is troublesome and relatively time consuming, on the part of both the patient and the ward staff. Normally, it requires three members of the ward staff to lift and turn such a patient. Since the urine bottles are open at one end, they allow bacteria to escape into the ambient air.

EP-A-0 209 508 discloses a more recent device for collecting and temporarily storing urine, comprising a urine tube connected to a collecting bag via a non-return valve. The urine tube is made of rigid plastic material and the connecting means, i.e. the one way valve means, include a float body, guided by a pin, said float body, being moveable between two positions, the valve being open in one position and closed in the other positions.

### The invention

The main object of the present invention is to provide an improved device of the kind set forth in the preamble of claim 1, such improved device having a much more reliable function and being very easy to fit to the urine outlet of persons of either sex, either bed-ridden, sitting or standing. Such an improved device has the combined features given in claim 1.

Patients who are obliged to lie in bed on their stomachs can also use the inventive with relative ease, irrespective of their sex. The presence of the valve means according to the invention eliminates the occurrence of urine vapour and obviates the risk of liquid running back to the urine tube, and also safe against the back flow of harmful bacteria from the urine collected in the bag. The novel urine collecting device is preferably intended for one-time use only and is also provided with an emptying mechanism, therewith facilitating the work of the nursing staff.

### Brief Description of the Drawing

So that the invention will be more readily understood and other features thereof made more apparent, the inventive device will now be described with reference to the accompanying drawings, in which
- Fig. 1: illustrates the inventive urine collecting device from above;
- Fig. 2: is a sectional view taken on the line A-A in Figure 1;
- Figure 3: is an axial sectional view of a component of the device illustrated in Figure 1;
- Figure 4: is a side view of the open end of the device;
- Figures 5 and 6: illustrate modified embodiments of the inventive device and correspond to the components illustrated in Figures 2 and 3.

### Description of Preferred Embodiments

The following description of the inventive urine collecting device is made with reference to the device when in use. Consequently all positional references are made with regard to the in-use position of the device.

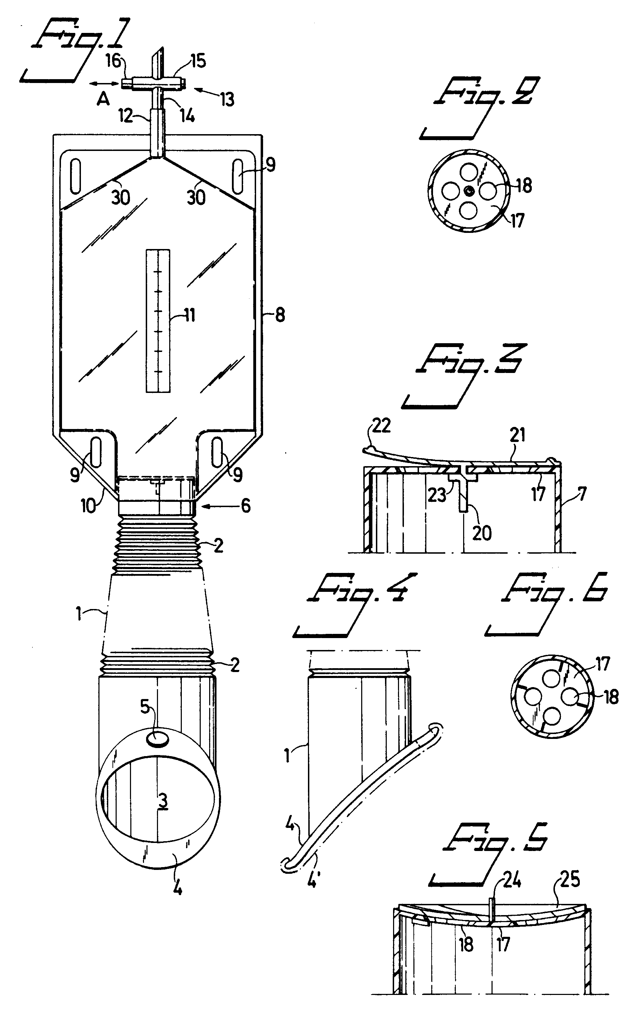

The illustrated urine collecting device comprises a urine tube 1 which is made of a relatively rigid plastics material and which has provided at one or more locations along its length bellows-like or pleated areas 2 which allow the tube to be bent or otherwise deflected plastically to selected angular positions. The mouth 3, or inlet aperture, of the urine tube 1 has an oval or circular configuration and preferably forms a so-called vagina-cup which can be fitted to the vagina of a female patient for instance. The inlet aperture, or mouth 3, has provided therearound a cuff 4 which extends approximately at right angles to the long axis of the urine tube 1. The inlet aperture 3 of the tube 1 may be fitted with a cover 4' as indicated by a chain line in Figure 4, so as to enable the inlet aperture to be closed. The reference 5 in Figure 1 illustrates a hole by means of which the device can be hung when not in use. The illustrated urine tube tapers symmetrically, e.g. may be conical so that the diameter of the tube at the end thereof incorporating the inlet aperture 3 is larger than the diameter of the opposite end of the tube. It will be seen from Figure 4 that the end of the tube 1 incorporating the inlet aperture 3 may be bevelled.

The end of the urine tube 1 distal from the inlet aperture 3 is joined to a valve housing, or valve means, generally referenced 6, which incorporates a circular valve body 7 which has a U-shape in longitudinal section and which is made of an inflexible or relatively inflexible plastics material.

The open end of a urine bag 8 is intended to be fitted around the valve housing 6, and thus also around the valve body 7, and to be bonded thereto, e.g heat welded thereto, so as to form an inseparable unit. The rim of the urine bag 8 preferably lies flush with the illustrated upper end surface of the valve housing 6, and a liquid-tight connection is created between the inner wall of the bag and the outer wall of the valve housing, in a manner hereinafter described.

The urine bag 8 together with its associated valve housing 6 is then inserted into the urine tube 1 with a press fit, so that the urine tube encloses the whole of the housing 6 or the major part of said housing. A liquid-tight join can be obtained between the urine tube on the one hand and the valve housing/urine bag on the other, for example by applying heat such as to cause the material of the valve housing/urine bag to flow out. It is also conceivable, when assembling the urine tube to the urine bag/valve housing unit to insert the tube 1 into the valve housing 6 so that the valve housing encloses the urine tube 1. An integrated leakage-free unit can also be achieved in this case, by applying heat to the various assembled components thereof.

The urine bag 8 is also made of plastics material, although of a thinner guage, and therewith a softer material than the material of the urine tube 1. As shown in Figure 1 that part of the urine-bag structure located adjacent the valve housing 6 has holes 9 provided therein for hanging the bag on a suitable support. The bag also has obliquely shaped corners 10 and a graduated scale 11. Holes 9 for hanging the bag onto a suitable support are also provided in the lower part of the urine bag structure. Located in the lower or bottom part of the urine bag construction is an opening for accommodating a urine drainage pipe 12. The pipe 12 co-acts with a valve arrangement, generally referenced 13, in which a further pipe 14 inserted into the pipe 12 is connected with a transverse pipe 15 in which a peg 16 can be moved axially, in the direction of the arrow A, in order to open or close the connection with the urine bag 8. It will be understood, however, that any suitable valve arrangement can be used to this end.

The bottom or lower part of the illustrated urine bag is also shaped obliquely (at 30) so that the bag slopes down towards the opening and thus towards the drainage pipe 12. This will ensure that the entire contents of the urine bag 8 will be emptied, without risk of any urine remaining in the bag.

In order to enable the inventive urine collecting device to be handled and carried without risk of urine flowing back from the urine bag and out through the urine tube 1, the device is provided with an improved valve housing or valve means 6, which functions in the manner of a non-return valve, thereby preventing said backflow of urine from the bag 8 and out through the urine tube 1. As before-mentioned, the inventive valve means can also be made impervious to harmful bacteria that are likely to develop in the urine contained in the bag, or to be carried by the urine from the body of the patient. The valve means can also be constructed to prevent urine vapour from escaping from the collecting device.

A first embodiment of such a valve means is illustrated in Figures 2 and 3.

The valve body 7 of the illustrated valve means has a valve bottom 17 in which four openings 18 are formed symmetrically around the centre axis of the bottom, these openings being of conical shape, i.e. tapering in a direction towards the urine pipe 1. In order, to enable the urine flowing through the urine tube 1 to pass readily into the valve means 6, the openings 18 have a relatively large size and are spaced from the wall of the valve means. A centrally located guide pin 20 is passed sealingly through an opening in the valve bottom 17 of the valve means 6. The guide pin 20 is firmly attached to a diaphragm 21, which is intended to lie against the surface of the valve means 6 facing inwardly towards the urine bag 8. The diaphragm 21 is dimensioned to cover the bottom 17 of the valve body 7, and extends out to the outer edge of the body 7, as illustrated in Figure 3. As illustrated, the diaphragm may be provided with an annular bead 22 which is operative in distributing liquid-pressure uniformly around the diaphragm. Thus, as urine flows through the tube 1 and passes through the holes 18, it exerts pressure on the diaphragm 21 which is therewith lifted or raised progressively in a direction away from the wall of the valve body 7 inwardly towards the centre, the extent to which the diaphragm is raised angularly in this way being contingent on the pressure and/or the quantity of the liquid present. This function of the valve means is illustrated in Figure 3. It will also be seen from Figure 3 that the guide pin 20 has a stop means 23 which prevents movement of the diaphragm 21 in the axial direction of the tube. The conicity of the holes 18 ensure optimum sealing of the diaphragm 21 against said holes.

Figures 5 and 6 illustrate a second embodiment of the valve means, in which the valve bottom 17 is slightly convex (this convex shape of the valve bottom 17 has been exaggerated in Figure 5.) In the case of the second embodiment, the bottom part of the valve body 7 facing towards the urine bag 8 has a centrally located guide pin 24 which preferably extends over the horizontal plane of the valve body. The guide pin 24 carries a diaphragm 25 which is dimensioned to cover effectively the drainage holes 18 in the valve bottom 17. The valve bottom 17 of this embodiment curves inwardly towards the imagined flow direction. The conicity of the drainage holes 18 ensures that the diaphragm will lie sealingly against the bottom of the valve means 6.

It has been found that, in addition to being inexpensive in manufacture, the valve means constructed in accordance herewith ensures that a highly efficient sealing effect is obtained. Tests were carried out on the inventive valve means, by causing liquid to run through the urine tube 1. The diaphragm 25 opened in response to the pressure exerted by the liquid, and liquid flowed down into the urine bag 8. The thin diaphragm 25 returned to its sealing abutment with the valve bottom as soon as all liquid had passed through. The liquid filled bag 8 was then turned upside down, i.e. so that the diaphragm was subjected to the weight of the overlying liquid. No leakage could be observed. It has been found that silicone rubber is a highly suitable material with regard to the diaphragm 25.

As beforementioned, the inventive urine collecting device is intended for one-time use only. The configuration of the inlet aperture 3 and the general construction of the device enables it to be used effectively by both men and women. The flexible zone or zones on the urine tube 1 enable the device to be used with the patient in a sitting, standing or lying position. Because of the construction of the valve means 6, the device can always be handled and carried without risk of urine flowing back through the urine tube 1. The urine bag 8 can be given a suitable volumetric capacity, such as to enable the device to be easily handled and carried. The urine is emptied through the drainage or emptying arrangement 13, and the device can be destroyed in a conventional manner. It has been calculated that the costs of purchasing and utilizing the inventive urine collected device are below the costs entailed by conventional handling of urine bottles and similar devices. The inventive urine collecting device is also advantageous from a medical aspect, since the exiting bacteria florance in the urine to the ambient air is optimally restricted.

The device according to the invention may be constructed to form an inseparable unit or assembly, or the urine bag and valve means can be joined to form a unit and the urine tube fitted to said unit by the nursing staff concerned.

## Claims

1. A disposable device for collecting and temporarily storing urine of an individual of either sex, comprising a tube (1) having urine inlet (3) and an opposite located urine outlet, and a urine collecting bag (8) having an inlet end and an openable and closable outlet end (12, 13) said bag having a bottom portion which converges in a direction towards the outlet end, and a non-return valve (6) located between said urine tube and said collecting bag, and serving as a connector means between said urine tube and said collecting bag, said urine tube tapering towards said valve means, wherein a proximal end of said valve means communicates with the urine tube, and a distal end communicates with the urine collecting bag, wherein the distal end of the valve body is embraced by the upper open end of the bag to establish communication between the valve means and said bag, wherein the valve means together with the urine collecting bag form a unit, wherein the outer surface of the distal end of the valve body lies sealingly against the inner wall of the urine tube in the region of its outlet, characterized by the combination of
a) said urine tube having provided at a location, means (2) enabling the tube to be bent plastically to a designed curved configuration, and
b) a valve means (6) which includes a valve body (7) which in longitudinal cross section is of U-shape, wherein the distal end (17) of the valve body fitted with a liquid and vapor impermeable diaphragm (21, 25) which deflects downwardly under the influence of liquid pressure directed towards the bag permitting liquid to flow into the bag, wherein the distal end of the valve body has arranged therein a perforated bottom against the undersurface of which the diaphragm seats in normal use of the device and the diaphragm being sufficiently resilient to return the diaphragm to a position sealingly against said perforations after liquid passes to seal the valve body against back-flow of liquid, gas or bacteria and wherein seating of the diaphragm on said bottom is guided by a centrally located guide means (20, 24) that holds the center of the diaphragm substantially in the center of the perforations.

2. A device according to claim 1, wherein perforations (18) in the bottom (17) of the valve means are arranged concentrically around the center of the bottom part, and wherein liquid entering from the urine tube is caused to flow through said perforations and cause the diaphragm to deflect downwardly while supported by the central guide means (20).

3. Device according to claim 1, wherein the bottom (17) of the valve means (6) curves inwardly towards the urine tube (1), and wherein a peg or pin (24) fixedly connected to the bottom part or the valve means forms a diaphragm guide means.

4. A device according to claim 1, wherein the diaphragm (21, 25) includes a circular bead (22) which is intended to distribute the weight acting on the diaphragm.

5. A device according to claim 4, wherein the circular bead (21) is located on or contiguous with the rim of the diaphragm (21, 25) and is made of the same material as said diaphragm.

6. A device according to claim 1, wherein the urine tube has bellows-like folds (2) along its lenght.

## Patentansprüche

1. Einmaleinrichtung zum Sammeln und vorübergehenden Speichern des Urins einer Person beliebigen Geschlechts, umfassend ein Rohr (1) mit einem Urineinlaß (3) und einem gegenüberliegend angeordneten Urinauslaß und einem Urinsammelbeutel (8), der ein Einlaßende und ein zu öffnendes und zu verschließendes Auslaßende (12, 13) aufweist, wobei dieser Beutel einen Bodenbereich hat, der in Richtung zum Auslaßende hin konvergiert, und ein Rückschlagventil (6), das zwischen dem Urinrohr und dem Sammelbeutel angeordnet ist und als Sammeleinrichtung zwischen dem Urinrohr und dem Sammelbehälter dient, wobei sich das Urinrohr zu der Ventileinrichtung hin verjüngt, wobei ein anliegendes Ende der Ventileinrichtung mit dem Urinrohr in Verbindung steht und ein abliegendes Ende mit dem Urinsammelbehälter in Verbindung steht, wobei das abliegende Ende des Ventilkörpers von dem oberen offenen Ende des Beutels umfaßt ist, um eine Verbindung zwischen der Ventileinrichtung und dem Beutel zu schaffen, wobei die Ventileinrichtung zusammen mit dem Urinsammelbehälter eine Einheit bildet, wobei die Außenseite des abliegenden Endes des Ventilkörpers an der Innenwand des Urinrohrs in der Nähe seines Auslasses dichtend anliegt, **gekennzeichnet** durch die Kombination von
a) das Urinrohr ist örtlich mit Mitteln (2) versehen, die ein plastisches Verbiegen des Rohres in eine gewünschte gekrümmte Form ermöglichen, und
b) eine Ventileinrichtung (6) mit einem Ventilkörper (7), der im Längsschnitt U-förmig ist, wobei das abliegende Ende (17) des Ventilkörpers mit einer flüssigkeits- und dampfundurchlässigen Membran (21, 25) versehen ist, die unter dem Einfluß eines gegen den Beutel gerichteten Flüssigkeitsdruckes nach unten ausweicht, so daß Flüssigkeit in den Beutel fließen kann, wobei in das abliegende Ende des Ventilkörpers ein gelochter Boden eingesetzt ist, an dessen Unterseite die Membran beim normalen Gebrauch der Einrichtung anliegt, und wobei die Membran ausreichend elastisch ist, damit die Membran in eine abdichtende Stellung an die Löcher zurückkehrt, um den Ventilkörper gegen ein Zurückfließen der Flüssigkeit, eines Gases oder von Bakterien abzudichten, und wobei der Sitz der Membran an dem besagten Boden durch ein zentral angeordnetes Führungsmittel (20, 24) geführt ist, welches das Zentrum der Membran im wesentlichen im Zentrum der Löcher hält.

2. Einrichtung nach Anspruch 1, wobei Löcher (18) im Boden (17) der Ventileinrichtung um das Zentrum des Bodenteils herum konzentrisch angeordnet sind und wobei die aus dem Urinrohr eintretende Flüssigkeit durch diese Öffnungen fließt und die Membran nach unten auslenkt, während sie von den zentralen Führungsmitteln (20) abgestützt ist.

3. Einrichtung nach Anspruch 1, wobei der Boden (17) der Ventileinrichtung (6) nach innen zu dem Urinrohr (1) hin gekrümmt ist und wobei ein an dem Bodenteil der Ventileinrichtung befestigter Zapfen oder Stift (24) ein Membran-Führungsmittel bildet.

4. Einrichtung nach Anspruch 1, wobei die Membran (21, 25) einen Ringwulst (22) aufweist, der dazu dient, das auf die Membran ausgeübte Gewicht zu verteilen.

5. Einrichtung nach Anspruch 4, wobei der Ringwulst (22) auf dem Rand der Membran (21, 25) angeordnet ist oder an diesen angrenzt und aus dem gleichen Material hergestellt ist wie die Membran.

6. Einrichtung nach Anspruch 1, wobei das Urinrohr entlang seiner Länge faltenbalgartige Falten (2) aufweist.

## Revendications

1. Dispositif pouvant être jeté et destiné à recueillir et à conserver temporairement de l'urine d'un individu de l'un ou l'autre sexe, comportant un tube (1) ayant une entrée (3) d'urine et une sortie d'urine située à l'opposé, et un sac (8) de collecte d'urine ayant une extrémité d'entrée et une extrémité (12, 13) de sortie pouvant être ouverte et être fermée, le sac ayant une partie de fond qui converge dans une direction dirigée vers l'extrémité de sortie, et une vanne (6) de non-retour située entre le tube d'urine et le sac de collecte, et servant en tant que moyen de connexion entre le tube d'urine et le sac de collecte, le tube d'urine étant de forme s'amincissant vers les moyens de vanne, une extrémité proximale des moyens de vanne communiquant avec le tube d'urine, et une extrémité distale communiquant avec le sac de collecte d'urine, l'extrémité distale du corps de vanne étant entourée par l'extrémité ouverte supérieure du sac de manière à établir une communication entre les moyens de vanne et le sac, les moyens de vanne formant avec le sac de collecte d'urine une unité, la surface extérieure de l'extrémité distale du corps de vanne s'appliquant à la paroi intérieure du tube d'urine de manière étanche dans la région de sa sortie, caractérisée par la combinaison de
a) le tube d'urine étant muni en un emplacement, de moyens (2) permettant au tube d'être courbé de manière plastique en une configuration incurvée conçue, et
b) des moyens (6) de vanne qui comportent un corps (7) de vanne qui en section transversale longitudinale a la forme d'un U, l'extrémité (17) distale du corps de vanne s'adaptant à une membrane (21, 25) imperméable aux liquides et aux vapeurs, qui fléchit vers le bas sous l'influence de la pression de liquide dirigée vers le sac en permettant au liquide de s'écouler dans le sac, l'extrémité distale du corps de vanne comportant en son sein un fond perforé contre la surface inférieure duquel la membrane repose en mode d'utilisation normale du dispositif et la membrane étant suffisamment élastique pour ramener la membrane en une position dans laquelle elle s'applique de manière étanche sur les perforations après que du liquide est passé de manière à rendre étanche le corps de vanne vis-à-vis d'un reflux de liquide, de gaz ou de bactéries, et la mise en assise de la membrane sur le fond est guidée par des moyens (20, 24) de guidage qui sont situés centralement et qui maintiennent le centre de la membrane sensiblement au centre des perforations.

2. Dispositif suivant la revendication 1, dans lequel des perforations (18) dans le fond (17) des moyens de vanne sont disposées concentriquement autour du centre de la partie de fond, et dans lequel du liquide entrant à partir du tube d'urine est amené à s'écouler par les perforations et fait en sorte que la membrane fléchisse vers le bas tout en étant supportée par les moyens (20) de guidage centraux.

3. Dispositif suivant la revendication 1, dans lequel le fond (17) des moyens (6) de vanne est incurvé vers l'intérieur en direction du tube (1) d'urine, dans lequel une broche (24) ou goupille connectée de manière fixe ou à la partie de fond des moyens de vanne forment des moyens de guidage de membrane.

4. Dispositif suivant la revendication 1, dans lequel la membrane (21, 25) comporte un bourrelet (22) circulaire qui est destiné à répartir le poids agissant sur la membrane.

5. Dispositif suivant la revendication 4, dans lequel le bourrelet (21) circulaire est situé sur le bord de la membrane (21, 25) ou à côté de celui-ci et est en le même matériau que la membrane.

6. Dispositif suivant la revendication 1, dans lequel le tube d'urine a des plis (2) en forme de soufflet sur sa longueur.
